# EUROPEAN PATENT APPLICATION

(11) **EP 4 524 126 A1**
(43) Date of publication of application: **19.03.2025**
(21) Application number: 23306528.3
(22) Date of filing: 15.09.2023
(51) Int. Cl.: C07C 67/56

(54) **METHOD FOR TREATING (METH)ACRYLATES BY TREATMENT WITH MONTMORILLONITE**

(71) Applicant: Bostik SA, 92700 Colombes (FR); ARKEMA FRANCE, 92700 Colombes (FR)
(72) Inventor: TCHAPLINSKI, Vladimir, 08193 BELLATERRA, BARCELONA (ES); DAVIN CARDONA, Laia, 08193 BELLATERRA, BARCELONA (ES); GONZALEZ GUERRERO, Maria Jose, 08193 BELLATERRA, BARCELONA (ES); CAMPANYÀ I LLOVET, Maria, 08193 BELLATERRA - BARCELONA (ES); NUNEZ, Sean, EXTON, 19341 (US)
(74) Representative: Arkema Patent

(57) **Abstract**

The present invention relates to a method for treating a (meth)acrylate, a (meth)acrylate obtained thereof, and its uses.

## Description

### Technical field

The present invention relates to a method for treating (meth)acrylates, (meth)acrylates obtained thereof, and their uses.

### Technical background

In order to broaden the application range of the cyanoacrylate materials and to improve their properties, cyanoacrylates may be mixed with (meth)acrylates. This allows customizing their material, chemical and physical properties.

However, as reported in the article entitled *"*The unintended consequences of trace impurities in polymer product development' from A. A. Parker (2008), the (meth)acrylates may comprise minor trace compounds, which are often inadvertently and unknowingly present/added to the raw material. Trace compounds may consist of trace additives, trace impurities, trace catalysts, trace by-products, ... Trace additives are compounds, which are purposely added to a formulation, such as inhibitors and stabilizers. Trace impurities are compounds, which are not purposely added to a formulation, such as inorganic elements and their derivatives. Trace impurities may occur due to transfer from one reactor to another, or due to vessel history (cross-contamination), etc ... Trace impurities may typically result from manufacturing processes of (meth)acrylates. For example, sulfur may derive from acids used in esterification process of (meth)acrylic acids with alcohol, or tin catalysts are common in the manufacture of urethane (meth)acrylates from isocyanate...

However, the grade of the materials used is of importance. Indeed, the grade of the materials, which is linked to their degree of purity, has an impact on their application range and on their performance. The trace impurities (trace by-products or trace catalysts...) in (meth)acrylates are undesirable and can affect material properties.

Besides, the presence of trace components in (meth)acrylates may prevent them for being used with other species due to the impact of those trace components to said species. For example, when mixed with cyanoacrylates, these trace compounds may initiate an unwanted, premature polymerization (curing of cyanoacrylate), which impair the performance of the material and reduce its shelf-life. Indeed, nucleophilic species (even at very small amounts such as ppm (by weight)) typically trigger cyanoacrylate polymerization, imparting the stability of the final composition.

In order to avoid this unwanted, premature polymerization, cyanoacrylates and (meth)acrylates may be provided as separate components. The user therefore extemporaneously obtains the composition by mixing both components together just before use. This product form is usually known as a 2K composition. Such extemporaneous composition has the drawback of not always being sufficiently homogeneously mixed, thereby leading to unreliable material, chemical and thermal properties. 2K compositions are also less easily dispensable than 1K composition, for the users, and are not suitable for many markets such where product application times are long while the mixed 2K product continues to react.

On the other hand, acidic species (acidic contaminants) as trace impurities present in (meth)acrylate may inhibit cyanoacrylate polymerization once mixed.

In order to avoid this unwanted premature polymerization, or unwanted inhibition, some (meth)acrylates have also been post-treated (after synthesis), in an attempt to remove at least part of the trace compounds (such as for example nucleophilic species, acidic species, etc). For example, the post-treatment may be selected from a water wash, an acidic wash, a neutralization wash, a brine wash or a stripping, distillation, chromatography. However, such post-treatments exhibit some drawbacks: it is not feasible or effective with all (meth)acrylates (for example high viscosity (meth)acrylate oligomers), it can lead to hydrolysis of esters (any aqueous treatment) which imparts its purity, it can form emulsions which are difficult to break, it requires additional steps (brine wash, drying, etc), it may increase the acidity of (meth)acrylate which impact the reactivity with cyanoacrylates, it generates aqueous wastes that need to be further treated...

Consequently, there is a need for providing a treatment method for obtaining a (meth)acrylate having a superior grade *i.e*., a (meth)acrylate being of high purity, that allows resolving at least part of the above-mentioned drawbacks.

More particularly, there is a need for providing a treatment method for obtaining (meth)acrylates having a reduced content of nucleophilic species.

More particularly, there is a need for providing a treatment method for obtaining (meth)acrylates having reduced content of inorganic trace compounds, or even reduced total content of metal and metalloid atoms.

There is also the need for providing a treatment method for limiting the compositional and manufacturing variability of (meth)acrylates, particularly in terms of trace compounds. There is also the need for providing (meth)acrylates exhibiting a superior compatibility with cyanoacrylates. There is also the need for providing a composition comprising (meth)acrylates and cyanoacrylates having a satisfactory stability over time, and therefore a satisfactory shelf-life. There is also the need for providing a composition comprising (meth)acrylate-functionalized compounds and cyanoacrylates, showing no unwanted, premature polymerization of cyanoacrylates.

### Summary of the invention

In a first aspect, the present invention relates to a method for treating a (meth)acrylate, said method comprising the step of treating the (meth)acrylate with a clay, wherein the clay is an acid-activated montmorillonite.

Preferably, the method comprises the following steps;
a) providing the (meth)acrylate;
b) mixing the (meth)acrylate with the clay;
c) optionally diluting the mixture of (meth)acrylate and the clay with a solvent;
d) treating the (meth)acrylate by leaving it in contact with the clay for a sufficient time;
e) separating the treated (meth)acrylate from the clay by filtration;
f) optionally eliminating the solvent from the treated (meth)acrylate; and
g) optionally storing the treated (meth)acrylate;

or the method comprises the following steps;
a') providing the (meth)acrylate;
b') optionally diluting the (meth)acrylate with a solvent;
c') treating the (meth)acrylate by filtrating it through at least one layer of the clay;
d') optionally eliminating the solvent from the treated (meth)acrylate; and
e') optionally storing the treated (meth)acrylate.

Preferably, the acid-activated montmorillonite is obtained from a natural montmorillonite having a 2:1 layer mineral and having two silica tetrahedral sheets bonded to a central alumina octahedral sheet.

Preferably, the acid-activated montmorillonite has a surface area BET from 150 to 380 m²g-¹.

Preferably, the acid-activated montmorillonite has a pore volume distribution (0-80 nm) from 0.20 to 0.55 ml. g⁻¹, a pore volume (0-24 nm) from 0.18 to 0.49 ml. g⁻¹, a pore volume (0-14 nm) from 0.13 to 0.43 ml. g⁻¹.

Preferably, the acid-activated montmorillonite has a S_{BET} from 150 to 380 m².g-¹, a number of sites from 0.13 to 0.32 mmol.g⁻¹, a number of sites from 0.50 to 1.40 µmol.m⁻².

Preferably, the (meth)acrylate comprises is selected from the group consisting of (meth)acrylic monomers, (meth)acrylic oligomers, and their mixtures thereof.

Preferably, the (meth)acrylate is selected from the group consisting of (meth)acrylate esters of aliphatic mono-alcohols, (meth)acrylate esters of alkoxylated aliphatic mono-alcohols, (meth)acrylate esters of aliphatic polyols, (meth)acrylate esters of alkoxylated aliphatic polyols, (meth)acrylate esters of aromatic ring-containing alcohols, (meth)acrylate esters of alkoxylated aromatic ring-containing alcohols, epoxy (meth)acrylates, polyether (meth)acrylates, urethane (meth)acrylates, polycarbonate (meth)acrylate), polyester (meth)acrylates, and mixtures thereof.

In particular, the (meth)acrylate is selected from the group consisting of: 1,3-butylene glycol di(meth)acrylate, 1,4-butanediol di(meth)acrylate, 1,6-hexanediol di(meth)acrylate, longer chain aliphatic di(meth)acrylates (such as those generally corresponding to the formula H₂C=CRC(=O)-O-(CH₂)ₘ-O-C(=O)CR'=CH₂, wherein R and R' are independently H or methyl and m is an integer of 8 to 24), alkoxylated (e.g., ethoxylated, propoxylated) hexanediol di(meth)acrylates, alkoxylated (e.g., ethoxylated, propoxylated) neopentyl glycol di(meth)acrylates, dodecyl di(meth) acrylates, cyclohexane dimethanol di(meth)acrylates, diethylene glycol di(meth)acrylates, dipropylene glycol di(meth)acrylates, alkoxylated (e.g., ethoxylated, propoxylated) bisphenol A di(meth)acrylates, ethylene glycol di(meth)acrylates, neopentyl glycol di(meth)acrylates, tricyclodecane dimethanol diacrylates, triethylene glycol di(meth)acrylates, tetraethylene glycol di(meth)acrylates, tripropylene glycol di(meth)acrylates, ditrimethylolpropane tetra(meth)acrylates, dipentaerythritol penta(meth)acrylates, alkoxylated (e.g., ethoxylated, propoxylated) pentaerythritol tetra(meth)acrylate, dipentaerythritol penta(meth)acrylates, pentaerythritol tetra(meth)acrylate, alkoxylated (e.g., ethoxylated, propoxylated) trimethylolpropane tri(meth)acrylates, alkoxylated (e.g., ethoxylated, propoxylated) glyceryl tri(meth)acrylates, trimethylolpropane tri(meth)acrylates, pentaerythritol tri(meth)acrylates, tris (2-hydroxy ethyl) isocyanurate tri(meth)acrylates, 2(2-ethoxyethoxy) ethyl (meth)acrylates, 2-phenoxyethyl (meth)acrylates, 3,3,5-trimethylcyclohexyl (meth)acrylates, alkoxylated lauryl (meth)acrylates, alkoxylated phenol (meth)acrylates, alkoxylated tetrahydrofurfuryl (meth)acrylates, caprolactone (meth)acrylates, cyclic trimethylolpropane formal (meth)acrylates, dicyclopentadienyl (meth)acrylates, diethylene glycol methyl ether (meth)acrylates, alkoxylated (e.g., ethoxylated, propoxylated) nonyl phenol (meth)acrylates, isobornyl (meth)acrylates, isodecyl (meth)acrylates, isooctyl (meth)acrylates, lauryl (meth)acrylates, methoxy polyethylene glycol (meth)acrylates, octyldecyl (meth)acrylates (also known as stearyl (meth)acrylates), tetrahydrofurfuryl (meth) acrylates, tridecyl (meth)acrylates, triethylene glycol ethyl ether (meth)acrylates, t-butyl cyclohexyl (meth)acrylates, dicyclopentadiene di(meth)acrylates, phenoxyethanol (meth)acrylates, octyl (meth)acrylates, decyl (meth)acrylates, dodecyl (meth)acrylates, tetradecyl (meth)acrylates, cetyl (meth)acrylates, hexadecyl (meth)acrylates, behenyl (meth)acrylates, diethylene glycol ethyl ether (meth)acrylates, diethylene glycol butyl ether (meth)acrylates, triethylene glycol methyl ether (meth)acrylates, dodecanediol di (meth)acrylates, dipentaerythritol penta/hexa(meth)acrylates, pentaerythritol tetra(meth)acrylates, alkoxylated (e.g., ethoxylated, propoxylated) pentaerythritol tetra(meth)acrylates, di-trimethylolpropane tetra(meth)acrylates, alkoxylated (e.g., ethoxylated, propoxylated) glyceryl tri(meth)acrylates, and tris (2-hydroxy ethyl) isocyanurate tri(meth)acrylates, and combinations thereof.

Preferably the (meth)acrylate is selected from the group consisting of oligomers such as epoxy (meth)acrylates, polyether (meth)acrylates, urethane (meth)acrylates, polycarbonate (meth)acrylate), polyester (meth)acrylates (including amine- and sulfide-modified derivatives thereof), and mixtures thereof.

Preferably, the (meth)acrylate does not comprise a cyano group.

Preferably, the solvent is a non-nucleophilic solvent. More preferably, the non-nucleophilic solvent is selected from the group consisting of aliphatic and aromatic hydrocarbons, ether, ester, ketone, nitrile, and mixtures thereof.

In a second aspect, the present invention concerns a (meth)acrylate obtained by the method disclosed herein, wherein the (meth)acrylate has a content of metal and metalloid atoms, lower than or equal to 200 ppm.

Preferably, the treated (meth)acrylate comprises less than 50 ppm of tin (Sn).

In another aspect, the present invention also concerns the use of the treated (meth)acrylate, in different applications, including in 3D printing, coating, sealant, adhesives, and electronics.

In another aspect, the present invention also relates to a curable composition comprising the treated (meth)acrylate.

In another aspect, the present invention also relates to the use of a clay being an acid-activated montmorillonite for treating a (meth)acrylate.

The inventors have surprisingly demonstrated that the treatment method according to the present invention allows obtaining (meth)acrylates having a superior grade i.e., (meth)acrylates being of high purity.

The treatment method according to the invention advantageously allows to reduce the content of nucleophilic species.

The treatment method according to the invention advantageously allows to reduce the content of trace compounds, in particular of inorganic trace compounds.

The treatment method advantageously allows reducing the total content of metal and metalloid atoms.

The inventors have also demonstrated that the treated (meth)acrylates exhibit a good shelf-life.

The inventors have also demonstrated that the treated (meth)acrylates exhibit a superior compatibility with cyanoacrylates, as they do not initiate any unwanted, premature polymerization of the cyanoacrylates upon mixing.

The present treatment method and the (meth)acrylate obtained thereof have at least one of the following advantages:
- Providing a universal method for treating (meth)acrylate ;
- Providing (meth)acrylate having a superior grade *i.e.,* (meth)acrylate being of high purity;
- Providing (meth)acrylate having a reduced total content of trace compounds, and more particularly a reduced content of inorganic trace compounds;
- Providing (meth)acrylate having a reduced content of nucleophilic species (such as for example phosphines, amines, strong bases, etc..) and strong acids;
- Providing (meth)acrylate having a reduced content of acidic species;
- Providing (meth)acrylate having a reduced total content of metal and metalloid atoms;
- Providing (meth)acrylate having a limited compositional and manufacturing variability in terms of trace compounds;
- Providing (meth)acrylate exhibiting a superior compatibility with cyanoacrylates;
- Providing a composition comprising (meth)acrylates and cyanoacrylates having a satisfactory stability over time, and therefore a satisfactory shelf-life;
- Limiting or preventing an unwanted, premature polymerization of cyanoacrylates, upon mixing with the (meth)acrylates; and
- Providing suitable materials for various applications using advance materials including in 3D printing, coating, sealant and electronics.

### Detailed description

The present treatment method corresponds to a purification treatment.

### Treatment method

In a first aspect, the present invention relates to a method for treating a (meth)acrylate, said method comprising the step of treating the (meth)acrylate with a clay, wherein the clay is an acid-activated montmorillonite.

This treatment step consists in bringing the (meth)acrylate in contact with the clay, thereby allowing advantageously the transfer of trace compounds (preferably inorganic trace compounds) from the (meth)acrylate to the clay. This treatment step may be implemented in different ways, for example but without being limited to them according to the first and second embodiments below.

In the first embodiment, the method preferably comprises:
a) providing the (meth)acrylate;
b) mixing the (meth)acrylate with the clay;
c) optionally diluting the mixture of (meth)acrylate and the clay with a solvent, preferably a non-nucleophilic solvent;
d) treating the (meth)acrylate by leaving it in contact with the clay for a sufficient time;
e) separating the treated (meth)acrylate from the clay by filtration;
f) optionally eliminating the solvent from the treated (meth)acrylate; and
g) optionally storing the treated (meth)acrylate.

In step a), the (meth)acrylate provided (provision step) is a raw (meth)acrylate. Then the raw (meth)acrylate is mixed with the clay (mixing step b). Optionally, the mixture may be diluted with a solvent (preferably a non-nucleophilic solvent), particularly for reducing its viscosity (dilution step c). The raw (meth)acrylate is left in contact with the clay for a sufficient time for allowing the transfer of trace compounds from the raw (meth)acrylate to the clay (treatment step d). After the treatment step, the mixture is filtrated, in order to separate the treated (meth)acrylate from the clay (step e). The treated (meth)acrylate is thus recovered. Optionally, the solvent (preferably non-nucleophilic solvent) present in the treated (meth)acrylate may be eliminated e.g., by evaporation (concentration step). Preferably, step f) is not optional when the solvent is a nucleophilic solvent.

In this first embodiment, the method comprises providing a raw (meth)acrylate as described herewith (provision step).

The method further comprises mixing the raw (meth)acrylate with a clay (mixing step b). The mixing step may last till a homogenous suspension is obtained. The mixing step may last from 5 min to 1 week, preferably from 30 min to 12h, more preferably from 1 h to 6 h. For example, the mixing step may last for 5 min. The mixing step may be carried out under agitation. Any suitable agitation devices may be used, including a magnetic agitation device or mechanical agitation device.

Optionally, the method may further comprise the step of diluting the mixture with a solvent (preferably non-nucleophilic solvent), particularly for reducing its viscosity (dilution step c). The diluted mixture may have a viscosity from 1 to 20 000 mPas (at 25°C), preferably from 1 to 500 mPas, more preferably from 1 to 250 mPas. The viscosity is a Brookfield viscosity measured according to the standard method ASTM method D-2393, using a Brookfield viscometer, at a temperature of 25°C.

The method further comprises treating the raw (meth)acrylate by leaving it in contact with the clay for a sufficient time, in order to allow the transfer of trace compounds from the raw (meth)acrylate to the clay. The raw (meth)acrylate may be left in contact with the clay from 5 min to 1 week, preferably from 1 hour to 12 hours, more preferably from 1 hour to 6 hours. For example, the treatment step may last for 1 hour. The treatment step is preferably carried under agitation. Any suitable agitation devices may be used, including a magnetic agitation device or a mechanical agitation device.

The method further comprises filtrating the mixture i.e., separating the treated (meth)acrylate from the clay by filtration. The mixture may be filtrated using any suitable filtration device. The filtration device may be selected from the group consisting of glass fiber filter, nylon fiber filter, polypropylene filter membrane, nutsche filter with or without filtration aids such as celite, magnesol or silica gel, frit funnel. For example, the filtration device may be a frit funnel.

Optionally, the method further comprises eliminating the solvent (preferably non-nucleophilic solvent) present in the treated (meth)acrylate e.g., by evaporation (concentration step). Preferably, step f) is not optional when the solvent is a nucleophilic solvent.

Optionally, the method further comprises storing the treated (meth)acrylate. The treated (meth)acrylate may be stored in a waterproof, gas-proof and/or opaque container.

In a second embodiment, the method according to the invention preferably comprises the following steps:
a') providing the (meth)acrylate;
b') optionally diluting the (meth)acrylate with a non-nucleophilic solvent;
c') treating the (meth)acrylate by filtrating it through at least one layer of the clay;
d') optionally eliminating the solvent (preferably non-nucleophilic solvent) from the treated (meth)acrylate; and
e') optionally storing the treated (meth)acrylate.

In step a'), the (meth)acrylate provided (provision step a') is a raw (meth)acrylate. The raw (meth)acrylate may be diluted with a solvent (preferably non-nucleophilic solvent), particularly for reducing its viscosity (dilution step b'). The raw (meth)acrylate is filtrated through a layer of clay, in order to bring the (meth)acrylate in contact with the clay, and for allowing advantageously the transfer of trace compounds from the raw (meth)acrylate to the clay (treatment step c'). Optionally, after the treatment step, the solvent (preferably non-nucleophilic solvent) present in the treated (meth)acrylate may be eliminated e.g., by evaporation (concentration step d'). Preferably, step d') is not optional when the solvent is a nucleophilic solvent.

In this second embodiment, the present treatment method comprises providing a raw (meth)acrylate as described herewith (provision step).

Optionally, the present treatment method may further comprise the step of diluting the (meth)acrylate with a solvent (preferably non-nucleophilic solvent), particularly for reducing its viscosity (dilution step). The diluted mixture may have a viscosity from 1 to 10 000 mPas (at 25°C), preferably from 1 to 500 mPas, more preferably from 1 to 250 mPas. The viscosity may be measured using Brookfield cup.

The treatment method further comprises filtrating the raw (meth)acrylate, optionally diluted raw (meth)acrylate, through at least one layer of clay (treatment step).

Optionally, the treatment method further comprises eliminating the solvent (preferably non-nucleophilic solvent) present in the treated (meth)acrylate e.g., by evaporation (concentration step). Preferably, step d') is not optional when the solvent is a nucleophilic solvent.

Optionally, the treatment method further comprises storing the treated (meth)acrylate. The treated (meth)acrylate may be stored in a waterproof, gas-proof and/or opaque container.

### Clays

The clay (clay mineral) is an acid-activated montmorillonite.

Montmorillonite is a known clay belonging to the following classification: aluminosilicates, phyllosilicates, smectites. As reported in the review entitled *"*Adsorption of a few heavy metals on natural and modified kaolinite and montmorillonite: A review" from K. G. Bhattacharyya et al., Advances in Colloid and Interface Science, 140 (2008) 114-131, natural (unmodified) montmorillonite has a 2:1 layer mineral and has two silica tetrahedral sheets bonded to a central alumina octahedral sheet. Montmorillonite (without interlayer material) has typically the following chemical formula:

(Si_{7.8}Al_{0.2})^{IV}(Al_{3.4}Mg_{0.6})^{IV}O₂₀(OH)₄ [V].

Its theoretical composition without the interlayer material is SiO₂ = 66.7 %, Al₂O₃ = 28.3 %, H₂O = 5 %. The net layer charge of the natural (unmodified) montmorillonite is -0.8 charge/unit cell.

Acid-activated montmorillonite (also known as acid-treated montmorillonite) is conventionally obtained by treating montmorillonite with inorganic acids of rather high concentration and usually at high temperature, followed by filtration and washing with water. The acids used may be HCl, sulfuric acid, nitric acid, boric acid, phosphoric acid, hydrofluoric acid, hydrobromic acid, ... Such treatment is conventionally known as acid activation, as reported in the above-mentioned review from K. G. Bhattacharyya *et al.* (2008) and in the article entitled "A study of the surface acidity of acid-treated montmorillonite clay catalysts" from U. Flessner et al., Journal of Molecular Catalysis A: Chemical, 168 (2001) 247-256. It is also disclosed in the article Applied Clay Science, vol. 80-81, August 2013, p.236-244, or even in US2006/046925.

The acid-activated montmorillonite may have a surface area BET from 150 to 380 m²g⁻¹. The surface area BET may be measured using standard BET method such as ASTM D3663.

The acid-activated montmorillonite may have a pore volume distribution (0-80 nm) from 0.20 to 0.55 ml. g⁻¹, a pore volume (0-24 nm) from 0.18 to 0.49 ml. g⁻¹, a pore volume (0-14 nm) from 0.13 to 0.43 ml. g⁻¹. The pore volume distributions may be measured using ASTM D3465.

The acid-treated montmorillonite may have a number of surface acidic sites as follows: S_{BET} from 150 to 380 m².g⁻¹ , a number of sites from 0.13 to 0.32 mmol.g⁻¹, a number of sites from 0.50 to 1.40 µmol.m⁻². The number of surface acidic sites is determined from the measurements of the adsorption of gaseous NH₃ at 353 K and P=38 Torr.

Suitable clays are commercially available under the K-series, including K5, K10, K20 and K30. As an example, the K10 grade is available under the tradenames S300-K10 from Clariant, Montmorillonite K10 from Thermo Fischer GmbH.

K5 has an apparent bulk density of about 600 g.I⁻¹, a surface area BET of about 200 m²g⁻¹, a pore volume (0-80 nm) of about 0.25 ml. g⁻¹, a pore volume (0-24 nm) of about 0.22 ml. g-¹, a pore volume (0-14 nm) of about 0.18 ml. g⁻¹. K5 has the following chemical composition (%): SiO₂ (65.0), AI₂O₃ (19.0), Fe₂O₃ (4.8), CaO (0.2), MgO (2.4), Na₂O (0.3), K₂O (1.5), loss on ignition (6.5), total (99.7). K5 has a number of surface acidic sites as follows: S_{BET} of about 200 m².g⁻¹, a number of sites of about 0.27 mmol.g⁻¹, a number of sites of about 1.35 µmol.m⁻².

K10 has an apparent bulk density of about 370 g.I⁻¹, a surface area BET of about 240 m²g⁻¹, a pore volume (0-80 nm) of about 0.36 ml. g⁻¹, a pore volume (0-24 nm) of about 0.30 ml. g⁻¹, a pore volume (0-14 nm) of about 0.26 ml. g-'. K10 has the following chemical composition (%): SiO₂ (73.0), Al₂O₃ (14.0), Fe₂O₃ (2.7), CaO (0.2), MgO (1.1), Na₂O (0.6), K₂O (1.9), loss on ignition (6.0), total (99.5). K10 has a number of surface acidic sites as follows: S_{BET} of about 240 m².g⁻¹, a number of sites of about 0.20 mmol.g⁻¹, a number of sites of about 0.83 µmol.m⁻².

K20 has an apparent bulk density of about 470 g.I⁻¹, a surface area BET of about 240 m²g⁻¹, a pore volume (0-80 nm) of about 0.39 ml. g⁻¹, a pore volume (0-24 nm) of about 0.32 ml. g⁻¹, a pore volume (0-14 nm) of about 0.30 ml. g-'. K20 has the following chemical composition (%): SiO₂ (75.0), Al₂O₃ (12.5), Fe₂O₃ (2.4), CaO (0.3), MgO (1.2), Na₂O (0.3), K₂O (1.5), loss on ignition (6.3), total (99.5). K20 has a number of surface acidic sites as follows: S_{BET} of about 240 m².g⁻¹, a number of sites of about 0.19 mmol.g⁻¹, a number of sites of about 0.79 µmol.m⁻².

K30 has an apparent bulk density of about 450 g.I⁻¹, a surface area BET of about 330 m²g⁻¹, a pore volume (0-80 nm) of about 0.50 ml. g⁻¹, a pore volume (0-24 nm) of about 0.44 ml. g⁻¹, a pore volume (0-14 nm) of about 0.38 ml. g⁻¹. K30 has the following chemical composition (%): SiO₂ (80.0), Al₂O₃ (10.0), Fe₂O₃ (1.8), CaO (0.2), MgO (1.0), Na₂O (0.3), K₂O (0.5), loss on ignition (6.0), total (99.8). K30 has a number of surface acidic sites as follows: S_{BET} of about 330 m².g⁻¹, a number of sites of about 0.18 mmol.g⁻¹, a number of sites of about 0.55 µmol.m⁻².

### (Meth)acrylate

As used herein, the term *"(meth)acrylate"* includes both acrylate and methacrylate.

Before treatment, the (meth)acrylate is called *"raw (untreated) (meth)acrylate"* herewith. After treatment, the (meth)acrylate is called *"treated (meth)acrylate"* herewith.

The (meth)acrylate typically comprises at least one (meth)acrylate group: 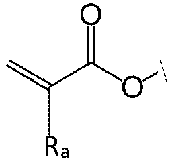 with Ra = H (acrylate), or Me (methacrylate)

The (meth)acrylate is preferably selected from (meth)acrylate monomers, (meth)acrylate oligomers, and their mixtures thereof.

The (meth)acrylate monomers may be selected from the group consisting of (meth)acrylate esters of aliphatic mono-alcohols, (meth)acrylate esters of alkoxylated aliphatic mono-alcohols, (meth)acrylate esters of aliphatic polyols, (meth)acrylate esters of alkoxylated aliphatic polyols, (meth)acrylate esters of aromatic ring-containing alcohols, (meth)acrylate esters of alkoxylated aromatic ring-containing alcohols, and mixtures thereof.

Illustrative examples of suitable (meth)acrylate monomers include (meth)acrylated mono- and polyols (polyalcohols) and (meth)acrylated alkoxylated mono-alcohols and polyols. The mono-alcohols and polyols may be aliphatic (including one or more cycloaliphatic rings) or may contain one or more aromatic rings (as in the case of phenol or bisphenol A). "Alkoxylated" means that the base mono-alcohol or polyol has been reacted with one or more epoxides such as ethylene oxide and/or propylene oxide so as to introduce one or more ether moieties (e.g., -CH₂CH₂-O-) onto one or more hydroxyl groups of the mono-alcohol or polyol, prior to esterification to introduce one or more (meth)acrylate functional groups. For example, the amount of epoxide reacted with the mono-alcohol or polyol may be from about 1 to about 30 moles of epoxide per mole of mono-alcohol or polyol. Examples of suitable mono-alcohols include, but are not limited to, straight chain, branched and cyclic C1-C54 mono-alcohols (which may be primary, secondary or tertiary alcohols). For instance, the mono-alcohol may be a C1-C7 aliphatic mono-alcohol. In another embodiment, the mono-alcohol may be a C8-C24 aliphatic mono-alcohol (e.g., lauryl alcohol, stearyl alcohol). Examples of suitable polyols include organic compounds containing two, three, four or more hydroxyl groups per molecule such as glycols (diols), e.g., ethylene glycol, 1,2- or 1,3-propylene glycol, or 1,2-, 1,3- or 1,4-butylene glycol, neopentyl glycol, trimethylolpropane, triethylolpropane, pentaerythritol, glycerol and the like.

Representative, but not limiting, examples of suitable (meth)acrylate monomers include: 1,3-butylene glycol di(meth)acrylate, 1,4-butanediol di(meth)acrylate, 1,6-hexanediol di(meth)acrylate, longer chain aliphatic di(meth)acrylates (such as those generally corresponding to the formula H₂C=CRC(=O)-O-(CH₂)ₘ-O-C(=O)CR'=CH₂, wherein R and R' are independently H or methyl and m is an integer of 8 to 24), alkoxylated (e.g., ethoxylated, propoxylated) hexanediol di(meth)acrylates, alkoxylated (e.g., ethoxylated, propoxylated) neopentyl glycol di(meth)acrylates, dodecyl di(meth) acrylates, cyclohexane dimethanol di(meth)acrylates, diethylene glycol di(meth)acrylates, dipropylene glycol di(meth)acrylates, alkoxylated (e.g., ethoxylated, propoxylated) bisphenol A di(meth)acrylates, ethylene glycol di(meth)acrylates, neopentyl glycol di(meth)acrylates, tricyclodecane dimethanol diacrylates, triethylene glycol di(meth)acrylates, tetraethylene glycol di(meth)acrylates, tripropylene glycol di(meth)acrylates, ditrimethylolpropane tetra(meth)acrylates, dipentaerythritol penta(meth)acrylates, alkoxylated (e.g., ethoxylated, propoxylated) pentaerythritol tetra(meth)acrylate, dipentaerythritol penta(meth)acrylates, pentaerythritol tetra(meth)acrylate, alkoxylated (e.g., ethoxylated, propoxylated) trimethylolpropane tri(meth)acrylates, alkoxylated (e.g., ethoxylated, propoxylated) glyceryl tri(meth)acrylates, trimethylolpropane tri(meth)acrylates, pentaerythritol tri(meth)acrylates, tris (2-hydroxy ethyl) isocyanurate tri(meth)acrylates, 2(2-ethoxyethoxy) ethyl (meth)acrylates, 2-phenoxyethyl (meth)acrylates, 3,3,5-trimethylcyclohexyl (meth)acrylates, alkoxylated lauryl (meth)acrylates, alkoxylated phenol (meth)acrylates, alkoxylated tetrahydrofurfuryl (meth)acrylates, caprolactone (meth)acrylates, cyclic trimethylolpropane formal (meth)acrylates, dicyclopentadienyl (meth)acrylates, diethylene glycol methyl ether (meth)acrylates, alkoxylated (e.g., ethoxylated, propoxylated) nonyl phenol (meth)acrylates, isobornyl (meth)acrylates, isodecyl (meth)acrylates, isooctyl (meth)acrylates, lauryl (meth)acrylates, methoxy polyethylene glycol (meth)acrylates, octyldecyl (meth)acrylates (also known as stearyl (meth)acrylates), tetrahydrofurfuryl (meth) acrylates, tridecyl (meth)acrylates, triethylene glycol ethyl ether (meth)acrylates, t-butyl cyclohexyl (meth)acrylates, dicyclopentadiene di(meth)acrylates, phenoxyethanol (meth)acrylates, octyl (meth)acrylates, decyl (meth)acrylates, dodecyl (meth)acrylates, tetradecyl (meth)acrylates, cetyl (meth)acrylates, hexadecyl (meth)acrylates, behenyl (meth)acrylates, diethylene glycol ethyl ether (meth)acrylates, diethylene glycol butyl ether (meth)acrylates, triethylene glycol methyl ether (meth)acrylates, dodecanediol di (meth)acrylates, dipentaerythritol penta/hexa(meth)acrylates, pentaerythritol tetra(meth)acrylates, alkoxylated (e.g., ethoxylated, propoxylated) pentaerythritol tetra(meth)acrylates, di-trimethylolpropane tetra(meth)acrylates, alkoxylated (e.g., ethoxylated, propoxylated) glyceryl tri(meth)acrylates, and tris (2-hydroxy ethyl) isocyanurate tri(meth)acrylates, and combinations thereof.

The (meth)acrylate oligomers may be selected from the group consisting of oligomers such as epoxy (meth)acrylates, polyether (meth)acrylates, urethane (meth)acrylates, polycarbonate (meth)acrylate), polyester (meth)acrylates (including amine- and sulfide-modified derivatives thereof), and mixtures thereof.

Exemplary polyester (meth)acrylates include the reaction products of acrylic or methacrylic acid or mixtures thereof with hydroxyl group-terminated polyester polyols. The reaction process may be conducted such that a significant concentration of residual hydroxyl groups remains in the polyester (meth)acrylate or may be conducted such that all or essentially all of the hydroxyl groups of the polyester polyol have been (meth)acrylated. The polyester polyols can be made by polycondensation reactions of polyhydroxyl functional components (in particular, diols) and polycarboxylic acid functional compounds (in particular, dicarboxylic acids and anhydrides). To prepare the polyester (meth)acrylates, the hydroxyl groups of the polyester polyols are then partially or fully esterified by reacting with (meth)acrylic acid, (meth)acryloyl chloride, (meth)acrylic anhydride or the like. Polyester (meth)acrylates may also be synthesized by reacting a hydroxyl-containing (meth)acrylate such as a hydroxyalkyl (meth)acrylate (e.g., hydroxyethyl acrylate) with a polycarboxylic acid. The polyhydroxyl functional and polycarboxylic acid functional components can each have linear, branched, cycloaliphatic or aromatic structures and can be used individually or as mixtures.

Examples of suitable epoxy (meth)acrylates include the reaction products of acrylic or methacrylic acid or mixtures thereof with glycidyl ethers or esters.

Exemplary polyether (meth)acrylate oligomers include, but are not limited to, the condensation reaction products of acrylic or methacrylic acid or mixtures thereof with polyetherols which are polyether polyols. Suitable polyetherols can be linear or branched substances containing ether bonds and terminal hydroxyl groups. Polyetherols can be prepared by ring opening polymerization of epoxides and other oxygen-containing heterocyclic compounds (e.g., ethylene oxide, 1,2-propylene oxide, butene oxide, tetrahydrofuran and combinations thereof) with a starter molecule. Suitable starter molecules include water, hydroxyl functional materials, polyester polyols and amines. Polyetherols may also be obtained by the condensation of diols such as glycols.

Urethane (meth)acrylates (sometimes also referred to as "polyurethane (meth)acrylates") capable of being used in the curable compositions of the present invention include urethanes based on aliphatic and/or aromatic polyester polyols, polyether polyols and polycarbonate polyols and aliphatic and/or aromatic polyester diisocyanates and polyether diisocyanates capped with (meth)acrylate end-groups.

In various embodiments, the urethane (meth)acrylates may be prepared by reacting aliphatic and/or aromatic polyisocyanates (e.g., diisocyanates, triisocyanates) with OH group terminated polyester polyols (including aromatic, aliphatic and mixed aliphatic/aromatic polyester polyols), polyether polyols, polycarbonate polyols, polycaprolactone polyols, polydimethysiloxane polyols, or polybutadiene polyols, or combinations thereof to form isocyanate-functionalized oligomers which are then reacted with hydroxyl-functionalized (meth)acrylates such as hydroxyethyl (meth)acrylate or hydroxypropyl (meth)acrylate to provide terminal (meth)acrylate groups. For example, the urethane (meth)acrylates may contain two, three, four or more (meth)acrylate functional groups per molecule. Other orders of addition may also be practiced to prepare the polyurethane (meth)acrylate, as is known in the art. For example, the hydroxyl-functionalized (meth)acrylate may be first reacted with a polyisocyanate to obtain an isocyanate-functionalized (meth)acrylate, which may then be reacted with an OH group terminated polyester polyol, polyether polyol, polycarbonate polyol, polycaprolactone polyol, polydimethysiloxane polyol, polybutadiene polyol, or a combination thereof. In yet another embodiment, a polyisocyanate may be first reacted with a polyol, including any of the aforementioned types of polyols, to obtain an isocyanate-functionalized polyol, which is thereafter reacted with a hydroxyl-functionalized (meth)acrylate to yield a polyurethane (meth)acrylate. Alternatively, all the components may be combined and reacted at the same time.

Any of the above-mentioned types of oligomers may be modified with amines or sulfides (e.g., thiols), following procedures known in the art. Such amine- and sulfide-modified oligomers may be prepared, for example, by reacting a relatively small portion (e.g., 2-15%) of the (meth)acrylate functional groups present in the base oligomer with an amine (e.g., a secondary amine) or a sulfide (e.g., a thiol), wherein the modifying compound adds to the carbon-carbon double bond of the (meth)acrylate in a Michael addition reaction.

Preferably, the (meth)acrylate monomer or oligomer does not comprise a cyano group. The raw (meth)acrylate may also comprise some trace compounds.

Conventional (meth)acrylate may comprise trace compounds, including organic trace compounds and inorganic trace compounds.

The raw (meth)acrylate may also comprise some nucleophilic species and/or acidic species, as contaminants.

Those contaminants/trace impurities may be obtained from manufacture processes of (meth)acrylate. Acidic species may be derived from strong acid catalyst, from side reactions during synthesis, from vessel contamination, from residual solvents, from unwanted byproducts, from degradation during storage, etc...

Conventional raw (meth)acrylate may comprise metal and metalloid atoms.

The metal and metalloid atoms are selected from the group consisting of: Li, Be, B, Na, Mg, Al, Si, K, Ca, Sc, Ti, V, Cr, Mn, Fe, Co, Ni, Cu, Zn, Ga, Ge, As, Rb, Sr, Y, Zr, Nb, Mo, Tc, Ru, Rh, Pd, Ag, Cd, In, Sn, Te, Cs, Ba, La, Hf, Ta, W, Re, Os, Ir, Pt, Au, Hg, TI, Pb, Bi, Ce, Pr, Nd, Sm, Eu, Gd, Tb, Dy, Ho, Er, Tm, Yb, Lu, Tu, U.

The (meth)acrylate (raw material) may comprise a total content of metal and metalloid atoms higher than 300 ppm, preferably higher than 400 ppm, and more preferably higher than 500 ppm.

The (meth)acrylate (raw material) may comprise atoms selected from the group consisting of: Li, Be, B, Na, Mg, Al, Si, P, S, K, Ca, Sc, Ti, V, Cr, Mn, Fe, Co, Ni, Cu, Zn, Ga, Ge, As, Se, Rb, Sr, Y, Zr, Nb, Mo, Tc, Ru, Rh, Pd, Ag, Cd, In, Sn, Te, I, Cs, Ba, La, Hf, Ta, W, Re, Os, Ir, Pt, Au, Hg, Tl, Pb, Bi, Ce, Pr, Nd, Sm, Eu, Gd, Tb, Dy, Ho, Er, Tm, Yb, Lu, Tu, and U.

The treatment method advantageously allows reducing the total content of metal and metalloid atoms between the raw (meth)acrylate and the treated (meth)acrylate from 2 to 20 folds.

The treated (meth)acrylate may have a total content of metal and metalloid atoms lower than or equal to 200 ppm, more preferably lower than or equal to 150 ppm.

The treatment method according to the invention advantageously allows reducing the total content of trace compounds, and more particularly inorganic trace compounds.

The treatment method according to the invention advantageously allows reducing the total content of nucleophilic species and/or acidic species.

The treatment method advantageously allows reducing trace level impurities, in particular pertaining to the manufacturing process of the raw (meth)acrylate.

The treatment method advantageously allows reducing the total content atom selected from Li, B, Na, Si, S, K, Ca, Br, Sn, I, and mixture thereof.

The treatment method advantageously allows reducing the total content of metal and metalloid atoms.

Tin may be present in the raw (meth)acrylate in its inorganic form as well as in the form of organotin compounds (OTC). However, many organotin compounds are toxic.

Advantageously, the treated (meth)acrylate may be substantially free of tin (Sn). It preferably comprises less than 50 ppm of Sn, even more preferably less than 20 ppm, and more preferably less than 5 ppm.

The content of atoms may be determined by spectroscopy, and more preferably using a Inductively Coupled Plasma Mass Spectrometer.

Suitable (meth)acrylate are commercially available under the following tradenames: SR150 from Sartomer (Ethoxylated bisphenol A dimethacrylate), SR339Y from Sartomer (2-phenoxyethyl acrylate), SR368 from Sartomer (tris (2-hydroxy ethyl) isocyanurate triacrylate), SR379 from Sartomer (glycidyl methacrylate), SR454 from Sartomer (ethoxylated trimethylolpropane triacrylate), SR495B from Sartomer (caprolactone acrylate), SR833S from Sartomer (tricyclodecanedimethanol diacrylate), SR834 from Sartomer (tricyclodecanedimethanol dimethacrylate).

### Solvent

The solvent is preferably a non-nucleophilic solvent.

The non-nucleophilic solvent may be selected from the group consisting of aliphatic and aromatic hydrocarbons, ether, ester, ketone, nitrile, and mixtures thereof. For example, the non-nucleophilic solvent may be acetonitrile, heptane, toluene, hexane, tetrahydrofuran (THF), methyl ethyl ketone (MEK).

The solvent is preferably a high-purity solvent.

Advantageously, the non-nucleophilic solvent comprises a reduced content (or is deprived) of nucleophilic species.

It can be selected from pure grade such as HPLC, ACS, USP, etc.

In the method according to the first embodiment, when the (meth)acrylate is mixed with the clay and solvent, the resulting mixture may comprise from 10 to 70% by weight, preferably from 20 to 60% by weight, more preferably from 30 to 50 % by weight of such solvent, by total weight of the mixture.

In the method according to the second embodiment, when the (meth)acrylate is mixed with the solvent, the resulting mixture (diluted (meth)acrylate) may comprise from 10 to 70 % by weight, preferably from 20 to 60% by weight, more preferably from 30 to 50 % by weight of such solvent, by total weight of the mixture.

### (Meth)acrylate obtained by the treatment method

The present invention also relates to the (meth)acrylate obtained by the method of treatment disclosed herein.

The treated (meth)acrylate preferably has a total content of metal and metalloid atoms lower than or equal to 200 ppm, more preferably lower than or equal to 150 ppm.

Advantageously, the treated (meth)acrylate may be substantially free of tin (Sn). It preferably comprises less than 50 ppm of Sn, even more preferably less than 20 ppm, and more preferably less than 5 ppm.

The treated (meth)acrylate preferably has a total content of Si of less than 100 ppm, preferably less than 50 ppm.

The treated (meth)acrylate preferably has a total content of S of less than 50 ppm, even preferably of less than 20 ppm.

### Curable compositions

The (meth)acrylate, obtained with the present treatment method, advantageously has a satisfactory purity, thereby corresponding to a material grade of high quality.

This (meth)acrylate is therefore suitable for use in a variety of formulations in different applications, including in 3D printing, coating, sealant, adhesives, and electronics. It can also be used for 1K system where trace contaminants would reduce the shelf life.

The present invention also relates to a curable composition comprising the (meth)acrylate obtained by the treatment method disclosed herein.

The curable composition may be typically prepared by mixing of the different ingredients, for example at 23°C.

The curable composition may be an adhesive, a coating, or a sealant.

The curable composition may comprise from 5% to 75% by weight, preferably from 10% to 25% by weight of the treated (meth)acrylate based on the total weight of the curable composition.

The curable composition preferably comprises at least one cyanoacrylate, in addition to the treated (meth)acrylate.

### Cyanoacrylates

As used herein, the term "cyanoacrylate" refers to an organic compound which contains a carbon-carbon double bond, wherein one carbon atom involved in the carbon-carbon double bond is substituted with a cyano (-CN) group and an ester group.

The cyanoacrylate may have the following chemical formula (VI): 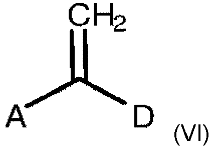 wherein:
A is CN; and
D is a carboxylic ester moiety CO₂R⁴,
wherein
R⁴ is selected from the group consisting of: C₁-C₁₈ linear and branched alkyl chains; terminally trimethylsilylated C₁-C₃ alkyl chains; -CH₂CF₃; -CH₂CF₂CF₃; -CH₂(CF₂)₂H; - CH₂(CF₂)₄H; -CH(CF₃)CH₃; allyl; propargyl; cyclohexyl; cyclohexenyl; methylcyclohexyl; methylcyclohexenyl; ethylcyclohexyl; ethylcyclohexenyl; furfuryl; phenylethyl; phenoxyethyl; an acrylic ester moiety of formula (VII): 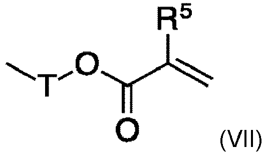
wherein
T is: (CH₂)_{z}, wherein the value of z is between 2 and 12 inclusive, a C₃-C₁₂ branched alkylene chain; cyclohexyl; bisphenyl, bisphenol, and R⁵ is H, Me, CN or CO₂R⁶, wherein R⁶ is a methyl or ethyl group;
or D is a group of formula (VIII)

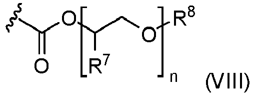

Wherein R⁷ is selected from the group consisting of H and Me, R⁸ is selected from the group consisting of Si(Me₃)₃ and C₁-C₆ linear and branched alkyl chains, and the value of n is from 1 to 3 inclusive.

When R⁵ is H or Me in formula (VII) then the ester group D of structure (VI) comprises an acrylate or methacrylate respectively and (VI) is then a compound with two "mixed" or "hybrid" functional groups (cyanoacrylate and acrylic).

When R⁵ is CN in (VII), then (VI) is a bis-cyanoacrylate. Preferred examples of cyanoacrylate esters with acrylic ester moieties, showing a hybrid or bis nature, include acryloylethyl cyanoacrylate, methacryloyldodecyl cyanoacrylate and 1,6-hexyl biscyanoacrylate.

The esters of general formula (VIII) can be defined generically as alkoxyalkyl or alkylsilyloxyalkyl esters, among which the following can be preferentially mentioned: 2-methoxyethyl, 2-ethoxyethyl, 2-propoxyethyl, 2-butoxyethyl, 2-isopropoxyethyl, 2-hexyloxyethyl, 2-amyloxyethyl, 2-ethoxybutyl, 2-methoxypropyl, and 2-(1-methoxy)propyl, trimethylsilyloxyethyl, hexamethyldisiloxanoxyethyl, among others. Other examples of alkoxyalkyl type monomers are described in U.S. Pat. No. 6,977,278. The alkoxylalkyl cyanoacrylates are generally low in odor and are non-staining and non-irritating. In certain preferred embodiments R⁷ is H and R⁸ is Me or Et and n is 1. Thus, in a preferred embodiment, the compound of general formula (VI) is 2-methoxyethyl cyanoacrylate or 2-ethoxyethyl cyanoacrylate. In another preferred embodiment, R⁷ is Me, R⁸ is Me, and n is 1. Thus, the compound of general formula (VI) may be 2-(1-methoxy)propyl cyanoacrylate.

Additional examples of suitable cyanoacrylates include, but are not limited to, alkyl and cycloalkyl α-cyanoacrylates such as methyl α-cyanoacrylate, ethyl α-cyanoacrylate, propyl α-cyanoacrylate, butyl α-cyanoacrylate, and cyclohexyl α-cyanoacrylate; alkenyl and cycloalkenyl α-cyanoacrylates such as allyl α-cyanoacrylate, methallyl α-cyanoacrylate, and cyclohexenyl α-cyanoacrylate; alkynyl α-cyanoacrylates such as propargyl α-cyanoacrylate; aryl α-cyanoacrylates such as phenyl α-cyanoacrylate and tolyl α-cyanoacrylate;heteroatom-containing α-cyanoacrylates such as methoxyethyl α-cyanoacrylate, ethoxyethyl α-cyanoacrylate, and furfuryl α-cyanoacrylate;and silicon-containing α-cyanoacrylates such as trimethylsilylmethyl α-cyanoacrylate, trimethylsilylethyl α-cyanoacrylate, trimethylsilylpropyl α-cyanoacrylate, and dimethylvinylsilylmethyl α-cyanoacrylate.

The cyanoacrylates defined by structure (VI) may be solid or liquid in physical form under normal ambient conditions, and may have functions that enable co-polymerisation and crosslinking. Solid monomers may be dissolved in liquid monomers to yield liquid formulations. In the context of the invention, within the monomers which are substantially odor-free or of low odour, are also included those monomers which are not lachrymatory, and those whose vapors tend not polymerize under ambient conditions to produce white deposits on the substrates to be bonded. These features are associated with low vapor pressures. Thus, for example, when R⁴ in the compound of general formula (VI) is 2-ethylhexyl, furfuryl, cyclohexyl, cyclohexenyl, terminally trialkylsilylmethyl (C₁-C₃ alkyl), or acryloylethyl cyanoacrylate, methacryloyldodecyl cyanoacrylate and 1 ,6-hexyl biscyanoacrylate the monomer is substantially odor-free or low odor in addition to the well-known alkoxyalkyl types. Monomers of structure (VI) bearing carboxylic esters further comprising structure (VII) have second polymerizable or copolymerizable functional groups and are thus considered to have dual or "mixed or hybrid" functionality, meaning more than one functional group is present, e.g., two CA groups in one molecule of structure (VI), or one CA functional group combined with one acrylic functional group in one molecule.

The cyanoacrylate is preferably selected from the group consisting of methyl cyanoacrylate, ethyl cyanoacrylate, n-propyl cyanoacrylate, iso-propyl cyanoacrylate, n-butyl cyanoacrylate, sec-butyl cyanoacrylate, iso-butyl cyanoacrylate, tert-butyl cyanoacrylate, n-pentyl cyanoacrylate, 1-methylbutyl cyanoacrylate, 1-ethylpropyl cyanoacrylate, neopentyl cyanoacrylate, *n*-hexyl cyanoacrylate, 1-methylpentyl cyanoacrylate, *n*-heptyl cyanoacrylate, *n*-octyl cyanoacrylate, 2-octyl cyanoacrylate, 2-ethylhexyl cyanoacrylate, *n*-nonyl cyanoacrylate, *n*-decyl cyanoacrylate, *n*-undecyl cyanoacrylate, *n*-dodecyl cyanoacrylate, n-octadecyl cyanoacrylate, allyl cyanoacrylate, cyclohexyl cyanoacrylate, 2-methoxyethyl cyanoacrylate, 2-ethoxyethyl cyanoacrylate, phenoxyethyl cyanoacrylate, 2-(1-alkoxy)propyl cyanoacrylate (for example 2-(1-methoxy)propyl cyanoacrylate), 2-(2'-alkoxy)-methoxyethyl-2"-cyanoacrylate (for example 2-(2'-methoxy)-methoxyethyl-2"-cyanoacrylate), 2-(2'-alkoxy)-ethoxyethyl-2"-cyanoacrylate (for example 2-(2'-methoxy)-ethoxyethyl-2"-cyanoacrylate, 2-(2'-ethoxy)-ethoxyethyl-2"-cyanoacrylate, 2-(2'-propyloxy)-ethoxyethyl-2"-cyanoacrylate, 2-(2'-butoxy)-ethoxyethyl-2"-cyanoacrylate, 2-(2'-pentyloxy)-ethoxyethyl-2"-cyanoacrylate or 2-(2'-hexyloxy)-ethoxyethyl-2"-cyanoacrylate), 2-(2'-alkoxy)-propyloxypropyl-2"-cyanoacrylate (for example 2-(2'-methoxy)-propyloxypropyl-2"-cyanoacrylate, 2-(2'-ethoxy)-propyloxypropyl-2"-cyanoacrylate, 2-(2'-propyloxy)-propyloxypropyl-2"-cyanoacrylate, 2-(2'-butyloxy)-propyloxypropyl-2"-cyanoacrylate, 2-(2'-pentyloxy)-propyloxypropyl-2"-cyanoacrylate, 2-(2'-hexyloxy)-propyloxypropyl-2"-cyanoacrylate), 2-(2'-alkoxy)-butyloxybutyl-2"-cyanoacrylate (for example 2-(2'-methoxy)-butyloxybutyl-2"-cyanoacrylate, 2-(2'-ethoxy)-butyloxybutyl-2"-cyanoacrylate, 2-(2'-butyloxy)-butyloxybutyl-2"-cyanoacrylate), 2-(3'-alkoxy)-propyloxyethyl-2"-cyanoacrylate for example 2-(3'-methoxy)-propyloxyethyl-2"-cyanoacrylate), 2-(3'-alkoxy)-butyloxyethyl-2"-cyanoacrylate (for example 2-(3' -methoxy)- butyloxyethyl-2"-cyanoacrylate), 2-(3'-alkoxy)-propyloxypropyl-2"-cyanoacrylate (for example 2-(3'-methoxy)-propyloxypropyl-2"-cyanoacrylate), 2-(3'-alkoxy)-butyloxypropyl-2"-cyanoacrylate (for example 2-(3'-methoxy)-butyloxypropyl-2"-cyanoacrylate 2-(2'-alkoxy)-ethoxypropyl-2"-cyanoacrylate (for example 2-(2'-methoxy)-ethoxypropyl-2"-cyanoacrylate), 2-(2'-alkoxy)-ethoxybutyl-2"-cyanoacrylate (for example 2-(2'-methoxy)-ethoxybutyl-2"-cyanoacrylate), trimethylsilyethyl cyanoacrylate, trimethylsilypropyl cyanoacrylate, trimethylsilyloxyethyl cyanoacrylate triethylsilyloxyethyl cyanoacrylate, phenylethyl cyanoacrylate, and mixtures thereof.

More preferably, the cyanoacrylate is a mono-functional cyanoacrylate, more preferably selected from 2-methoxyethyl cyanoacrylate, methyl cyanoacrylate, ethyl cyanoacrylate, n-propyl cyanoacrylate, n-heptyl cyanoacrylate, n octyl cyanoacrylate, iso-propyl cyanoacrylate or mixtures thereof.

The curable composition preferably comprises from 25% to 90% by weight of cyanoacrylate (different from the treated (meth)acrylate), more preferably from 60 to 80% by weight based on the total weight of the curable composition.

The curable composition may also comprise the following compounds: acid inhibitors, radical stabilizers, thickeners, tougheners, thixotropic agent, fillers, pigments, plasticizers, accelerators, adhesion promoters, and mixtures thereof.

### Acidic inhibitors

The curable composition may comprise an acidic inhibitor.

The acid inhibitors are inhibitors of the anionic polymerization. They may be selected from the group consisting of Bronsted acids, Lewis acids, acids provided from an acidic ion exchange material or photoacids obtained from photoacid generators (also known as PAGs), and mixtures thereof; preferably from Bronsted acids, Lewis acids, and mixtures thereof. The acid inhibitors are preferably selected from the group consisting of methanesulfonic acid, p-toluenesulfonic acid, p-toluenesulfonic acid anhydride, hydrofluoric acid, boron trifluoride, boron trifluoride etherate complex, boron trifluoride dihydrate, sulfur dioxide, sulfur trioxide, tin (IV) chloride, iron (III) chloride, citric acid, trimethylsilyl triflate, and mixtures thereof; more preferably from methanesulfonic acid, hydrofluoric acid, boron trifluoride, boron trifluoride etherate complex, sulfur dioxide, and mixtures thereof.

The curable composition may comprise from 0.0003 to 0.1 %, preferably from 0.001 to 0.05 %, more preferably from 0.0015 to 0.02 % by weight, of an acidic inhibitor, by total weight of the curable composition.

### Radical stabilizers

The expressions "radical stabilizer" and "radical stabilizing agent" are presently used interchangeably.

The curable composition may comprise a radical stabilizer.

The radical stabilizer is a free radical polymerization inhibitor. It is preferably selected from the group consisting of hindered phenolic or polyphenolic compounds; preferably from hydroquinone, hydroquinone monomethyl ether, mono-tertiary-butyl hydroquinone, 2,5-ditertiary-butyl-hydroquinone, p-methoxyphenol, hydroxyanisole, butylated hydroxyanisole, hydroxyanisol butyl ether, 2,6-di-tert-butyl-p-cresol, 2,2'-methylene-bis-(6-tert-butyl-4-methylphenol), p-tert-butyl catechol, 1,3,5-trimethyl-2,4,6-tris(3,5-di-tert-butyl-4-hydroxybenzyl) benzene, hydroxytoluene butyl ether, and mixtures thereof; more preferably from hydroquinone monomethyl ether, 2,2'-methylene-bis-(6-tert-butyl-4-methylphenol), and mixtures thereof.

The curable composition may comprise from 0.01 to 0.7 % by weight, preferably from 0.01 to 0.5% by weight, more preferably from 0.01 to 0.4 % by weight, of a radical stabilizer, by total weight of the curable composition.

### Thickeners

The expressions "thickeners" or "thickening agents" are presently used interchangeably.

The curable composition may comprise a thickener.

Thickeners are particularly suitable for controlling and/or increasing the viscosity of the cyanoacrylate component, considering that cyanoacrylate-based monomers usually have a water-like viscosity at ambient temperature. A suitable thickener or thickening agent for the first part of the composition can be selected from those that are compatible with the host monomers. Examples of such thickeners include poly(meth)acrylates, acylated cellulose polymers (for example, cellulose acetate), polyvinyl acetates, partially hydrolysed polyvinyl acetates, polyvinylpyrrolidones, polyoxylates, polycaprolactones, polycyanoacrylates, vinyl acetate copolymers (for example, with vinyl chloride), copolymers of (meth)acrylates with butadiene and styrene, copolymers of vinyl chloride and acrylonitrile, copolymers of ethylene and vinyl acetate, poly[butyleneterephthalate-co-polyethyleneglycolterephthalate, copolymers of lactic acid and caprolactone, and mixtures thereof.

These thickeners are well known to the skilled in the art and have been described in the prior art. Preferably, the thickener is selected from the group consisting of poly(meth)acrylates, polymethyl(meth)acrylate, polyvinylpyrrolidones, polyvinyl acetates, partially hydrolysed polyvinyl acetates, vinyl acetate copolymers, acrylated cellulose polymers, and mixtures thereof. A suitable thickener for the cyanoacrylate component may be, for example, polymethylmethacrylate (for example Degacryl^{®} M 449, Evonik), copolymers of vinyl acetate and vinyl alcohol (for example Levamelt^{®} 900, Lanxess), copolymers of vinyl chloride and vinyl acetate (for example, Vinnol^{®} H 40-60, Wacker), copolymers of ethylene, vinyl acetate, and esters or partial esters of maleic acid (for example, Vamac^{®} G, DuPont), and mixtures thereof.

The curable composition may comprise from 2 to 10 % by weight, preferably from 3 to 8 % by weight, more preferably from 4 to 7 % by weight, of a thickener, by total weight of the curable composition.

### Touaheners

The expressions "tougheners" or "toughening agents" are presently used interchangeably.

The curable composition may comprise a toughener.

The toughener may be selected from the group consisting of block copolymers (for example, polymethylmethacrylate-co-polybutylacrylate-co-polymethylmethacrylate), elastomeric rubbers, elastomeric polymers, liquid elastomers, polyesters, acrylic rubbers, butadiene/acrylonitrile rubber, Buna rubber, polyisobutylene, polyisoprene, natural rubber, synthetic rubber (for example, styrene/butadiene rubber (SBR)), polyurethane polymers, ethylene-vinyl acetate polymers, fluorinated rubbers, isoprene-acrylonitrile polymers, chlorosulfonated polyethylenes, homopolymers of polyvinyl acetate, block copolymers, core-shell rubber particles (for example, commercially available from Arkema under the trade name Clearstrength^{®} XT100), and mixtures thereof.

The curable composition may comprise from 1 to 20 % by weight, preferably from 5 to 12 % by weight, of a toughener, by total weight of the curable composition.

### Thixotropic agent

The curable composition may comprise a thixotropic agent.

The thixotropic agent may be an organic thixotropic agent, an inorganic thixotropic agent; preferably a thixotropic agent selected from the group consisting of hydrogenated castor oil, hydrogenated castor oil modified by reaction with an amine, polyamides, silica, and mixtures thereof.

The silica may be fumed silica; preferably silica may be selected from the group consisting of fumed silica, hydrophobic fumed silica, hydrophilic fumed silica and precipitated silica, and mixtures thereof; more preferably from hydrophobic fumed silica. Hydrophobic fumed silica may also act as a filler. A hydrophobic fumed silica is commercially available under the commercial denomination Aerosil^{®} R202 from Evonik.

The curable composition may comprise from 0.25 to 10 % by weight, preferably from 2.5 to 8 % by weight, more preferably from 3 to 6 % by weight, of a thixotropic agent, by total weight of the curable composition.

### Fillers and pigments

In addition to hydrophobic fumed silica if present, the curable composition may comprise other fillers and pigments; preferably acid-treated fillers and pigments; more preferably organo-silicone modified surface-treated pigments and fillers.

The filler may also be a non-soluble filler; preferably a filler selected from the group consisting of low density polyethylene powder, polyvinylidene fluoride (PVDF), polytetrafluoroethylene (PTFE), and mixtures thereof.

The curable composition may comprise from 2 to 4 % by weight of fillers, by total weight of the cyanoacrylate component. Alternatively, or cumulatively, if present, the additional component may comprise from 3 to 10% by weight, preferably from 4 to 9% by weight (alternatively from 0.5 to 3 % by weight, preferably from 12 % by weight) of fillers, by total weight of the curable composition.

### Plasticizers

The curable composition may comprise a plasticizer.

The plasticizer may be selected from the group consisting of esters of C2-C4 carboxylic acids with C2-C5 alkyl alcohols having 2-4 hydroxyl groups (for example, ethylene glycol, propylene glycol, glycerine, trimethylolpropane, or pentaerythritol), optionally ethoxylated; preferably from acetates of C2-C5 alkyl alcohols having 2-4 hydroxyl groups, optionally ethoxylated; and more preferably from acetates of C2-C3 alkyl alcohols having 2-3 hydroxyl groups (for example, glycerine triacetate, ethylene glycol diacetate, and mixtures thereof).

The plasticizer may also be selected from the group consisting of phthalates (for example, dioctyl phthalate), trimellitates (for example, triocctyl trimellitate), adipates (for example, dimethyl adipate), benzoates (for example, diethylene glycol dibenzoate, isodecyl benzoate, and mixtures thereof), and mixtures thereof.

In some embodiments, the plasticizer may be a plasticizer of low hydrophilicity, for example dibutyl sebacate, acetyl tributyl citrate, isodecyl benzoate, and mixtures thereof.

The curable composition may comprise from 10 to 20 % by weight, preferably from 15 to 18 % by weight, of plasticizer, by total weight of the curable composition.

### Accelerators

The curable composition may comprise an accelerating agent.

The expressions "accelerator" or "accelerating agent" are presently used interchangeably.

The accelerator may be selected from the group consisting of crown ethers (for example, 15-crown-6 ether, 15-crown-5 ether, 18-crown-6 ether, dibenzo-18-crown-6 ether, and mixtures thereof), cyclodextrins, silylated crown ethers, calixarenes tetra-t-butyl esters, dimethyl ethers of PEG 400, PEG 500, PEG 600 and PEG 1000, and mixtures thereof; preferably the accelerator is a crown ether; more preferably the accelerator is selected from the group consisting of 18-crown-6 ether, dibenzo-18-crown-6 ether and mixtures ; still more preferably the accelerator is dibenzo-18-crown-6.

The curable composition may comprise from 0.01 to 0.8 % by weight, preferably from 0.05 to 0.5 % by weight, of an accelerator, by total weight of the curable composition.

### Adhesion promoters

The curable composition may comprise an adhesion promoting agent.

The expression "adhesion promoter" and "adhesion promoting agent" are presently used interchangeably.

The curable composition may comprise an adhesion promoter for glass, ceramics, porcelain, plastics and/or metal such as, for example, alkoxysilane compounds, citric acid, lithium tetrafluoroborate, lithium hexafluorophosphate, an aromatic carboxylic acid or anhydride or an α-substituted acrylic acid, optionally in form of anhydride. Among the acids and anhydrides can be mentioned trimellitic acid, trimellitic anhydride, pyromellitic acid, pyromellitic anhydride, 3,3',4,4'-benzophenonetetracarboxylic acid dianhydride, itaconic acid, itaconic anhydride, and 3-buten-1,2,3-tricarboxylic acid.

The curable composition may comprise from 0.05 to 0.3 % by weight, preferably from 0.06 to 0.08 % by weight, of an adhesion promoter, by total weight of the curable composition.

The curable composition may be obtained by mixing a treated (meth)acrylic resin as defined above and additional ingredients, such as for example those defined above, in particular with cyanoacrylate(s).

The curable composition has advantageously a satisfactory shelf-life.

### Uses

The present invention also concerns the use of a clay being an acid-activated montmorillonite for treating a (meth)acrylate.

The present invention also concerns the use of a clay being an acid-activated montmorillonite for purifying a (meth)acrylate.

The description and preferred embodiments disclosed above for both the acid-activated montmorillonite and the (meth)acrylate, apply herein for the mentioned use.

More preferably, the use of the clay being an acid-activated montmorillonite advantageously allows reducing the total content of metal and metalloid atoms.

The ranges disclosed in this description include both the lower and the upper limit thereof. For example, the expressions "ranging from x to y" or "between x and y" comprises the limits x and y.

### Examples

Ethoxylated trimethylolpropane triacrylate (SR454): commercialized by ARKEMA 2-Methoxyethyl Cyanoacrylate (MECA) : commercialized by CMC Mixture SR833 tricyclodecanedimethanol diacrylate/ SR368 tris(2-hydroxyethyl)isocyanurate triacrylate (7/3)
SR939 (phenoxyethylacrylate) commercialized by ARKEMA
CN1964 (aliphatic urethane dimethacrylate) commercialized by ARKEMA

### Example 1: comparative - Use of SR454

2g of SR454 and 8g of 2-Methoxyethyl Cyanoacrylate (MECA) have been mixed at room temperature (23°C). Polymerization of the mixture has been observed after 3 seconds.

### Example 2: process of purification of SR454

To a sample of SR454 (100 g) was added acid-treated clay (6 g, Clariant K10-S300) and the sample was mixed vigorously with an overhead agitator at room temperature (23°C) for 3 hours. After the 3 hour stirring period, the agitation was removed and the suspension allowed to settle for an additional 1 hour. The sample of SR454, then clear from settling, was decanted and gravity filtered in stages with paper filters starting at 40 micron nominal porosity, followed by 15 micron nominal porosity, and finally 1 micron nominal porosity to yield a clear treated sample of SR454 (89 gram yield).

### Example 3 : analytical testing of acrylate pre and post clay treatment for SR454

### ICP-MS spectroscopy

The instruments used is an Agilent Inductively Coupled Plasma Mass Spectrometer (ICP-MS) Model 7900, a Milestone Ultrawave microwave digester and Mettler Toledo XPE205DR analytical balance. Samples of 0.5 grams was digested in a mixture of concentrated HNO₃, HCl, and HF in a microwave oven with digestion blanks in parallel. The resulting digestions were diluted with 1% HNO₃ (v/v). Instrument reporting was obtained from molar response curve and atomic weight.

Two samples were analyzed: the untreated SR454 (raw (meth)acrylate) and the SR454 obtained after purification treatment of example 2 (treated).

All the atoms were analyzed except H, C, N and O. Only part of them is reproduced in the following table. The total of inorganic elements was also determined.

| | Untreated SR454 (commercialized by Arkema) | Treated SR454 (from example 2) |
|---|---|---|
| Na (ppm) | 1.4 | 0 |
| Si (ppm) | 111.6 | 4.7 |
| Ca (ppm) | 26.9 | 0 |
| | | |
| Other cumulative inorganic (ppm) | 1.3 | 1.2 |
| Total inorganic atoms (except H, N, C and O) (ppm) | 142.4 | 6.0 |

According to the analysis, the total content of inorganic atoms (excluding H, C, N and O) is advantageously lower for the treated SR454 compared to the untreated SR454 (raw).

### Example 4 : Use of the purified SR 454

2g of purified SR454 (resulting from example 2) and 8g of 2-Methoxyethyl Cyanoacrylate (MECA) have been mixed at room temperature (23°C). No polymerization has been observed after 24h at room temperature (23°C).

The treated SR454 thus advantageously can be stored with a MECA without premature polymerization, compared to example 1 which uses an untreated SR454.

### Example 5 : comparative - use of a mixture of SR833 (tricyclodecanedimethanol diacrylate)/SR368 (tris(2-hydroxyethyl)isocyanurate triacrylate) (7/3)

2g of the mixture SR833/SR368 (7/3) and 8g of 2-Methoxyethyl Cyanoacrylate (MECA) have been mixed at room temperature (23°C). Polymerization of the mixture has been observed after 3 seconds.

### Example 6 : process of purification of a mixture of SR833 (tricyclodecanedimethanol diacrylate)/SR368 (tris(2-hydroxyethyl)isocyanurate triacrylate) (7/3) - analytical testing

To a sample of a mixture SR833/SR368 (7/3) (100 g) was added 20 grams of toluene to reduce viscosity. To the resulting solution was added acid-treated clay (6 g, Clariant K10-S300) and the sample was mixed vigorously with an overhead agitator at room temperature (23°C) for 3 hours. After the 3 hour stirring period, the agitation was removed and the suspension allowed to settle for an additional 1 hour. The sample, then clear from settling, was decanted and gravity filtered in stages with paper filters starting at 40 micron nominal porosity, followed by 15 micron nominal porosity, and finally 1 micron nominal porosity. The solution was then stripped from toluene under reduced pressure to yield a clear treated sample of the mixture SR833/SR368 (87 gram yield).

The untreated and the treated mixture SR833/SR368 (from example 6) were analyzed in conformity with the test method of example 3.

The results are as follows:

| | Untreated mixture SR833/SR368 (7/3) | Treated mixture SR833/SR368 (7/3) (from example 6) |
|---|---|---|
| Na | 0.0 | 17.1 |
| Si | 41.7 | 62.4 |
| P | 13.0 | 8.6 |
| S | 120.9 | 0 |
| K | 4.5 | 2.2 |
| Br | 9.4 | 1.1 |
| I | 14.2 | 5.4 |
| Other cumulative inorganics (ppm) | 1.7 | 1.0 |
| Total inorganic atoms (except H, N, C and O) (ppm) | 205.4 | 98.5 |

According to the analysis, the total content of inorganic atoms (excluding H, C, N and O) is advantageously lower for the treated mixture compared to the untreated mixture (raw).

### Example 7 : Use of the purified mixture SR833/SR368 (7/3)

2g of purified mixture (resulting from example 6) and 8g of 2-Methoxyethyl Cyanoacrylate (MECA) have been mixed at room temperature (23°C). No polymerization has been observed after 24h at room temperature (23°C).

The treated mixture of example 6 thus advantageously can be stored with a MECA without premature polymerization, compared to example 5 which uses an untreated mixture.

### Example 8: comparative - Use of SR339 (2-phenoxyethyl acrylate)

2g of SR339 and 8g of 2-Methoxyethyl Cyanoacrylate (MECA) have been mixed at room temperature (23°C). Polymerization of the mixture has been observed after 3 seconds.

### Example 9: Process of purification of SR339 (2-phenoxyethyl acrylate)

A 100 gram sample of SR339 was purified in the same manner as SR454 in example 2 to yield a clear treated sample in a 92 gram yield.

The untreated and the treated SR339 (from example 9) were analyzed in conformity with the test method of example 3.

The results are as follows:

| | Untreated SR339 (commercialized by Arkema) | Treated SR339 (from example 9) |
|---|---|---|
| Si | 20.0 | 0.0 |
| P | 2.0 | 4.0 |
| S | 310.0 | 0.0 |
| Ca | 0.0 | 15.0 |
| Br | 1.0 | 0.2 |
| Other cumulative inorganics (ppm) | 2.1 | 1.0 |
| Total inorganic atoms (except H, N, C and O) (ppm) | 335.1 | 20.2 |

### Example 10 : Use of the purified SR 339

2g of purified SR339 (resulting from example 9) and 8g of 2-Methoxyethyl Cyanoacrylate (MECA) have been mixed at room temperature (23°C). No polymerization has been observed after 24h at room temperature (23°C).

The treated SR339 of example 9 thus advantageously can be stored with a MECA without premature polymerization, compared to example 8 which uses an untreated SR339.

### Example 11: comparative - Use of CN1964 (aliphatic urethane dimethacrylate)

2g of CN1964 and 8g of 2-Methoxyethyl Cyanoacrylate (MECA) have been mixed at room temperature (23°C). Polymerization of the mixture has been observed after 5 seconds.

### Example 12: Purification of CN1964 (aliphatic urethane dimethacrylate)

A 100 gram sample of CN1964 was diluted with 30 grams of toluene and was purified in the same manner as the mixture of methacrylates in example 6 to yield a clear treated sample of CN1964 in an 89 g yield.

The untreated and the treated CN1964 (from example 12) were analyzed in conformity with the test method of example 3.

The results are as follows:

| | Untreated CN1964 (commercialized by Arkema) | Treated CN1964 (from example 12) |
|---|---|---|
| B | 0.0 | 13.3 |
| Na | 0.5 | 0.1 |
| Si | 91.3 | 75.0 |
| P | 1.5 | 9.6 |
| S | 41.1 | 38.2 |
| K | 2.4 | 4.0 |
| Ca | 27.1 | 0.0 |
| Br | 13.1 | 7.4 |
| Sn | 36.7 | 3.2 |
| I | 12.4 | 9.2 |
| Other cumulative inorganics (ppm) | 2.4 | 3.5 |
| Total inorganic atoms (except H, N, C and O) (ppm) | 228.5 | 163.5 |

### Example 13 : Use of the purified CN1964

2g of purified CN1964 (resulting from example 10) and 8g of 2-Methoxyethyl Cyanoacrylate (MECA) have been mixed at room temperature (23°C). No polymerization has been observed after 24h at room temperature (23°C).

The treated CN1964 of example 12 thus advantageously can be stored with a MECA without premature polymerization, compared to example 11 which uses an untreated CN1964.

## Claims

1. A method for treating a (meth)acrylate, said method comprising the step of treating the (meth)acrylate with a clay, wherein the clay is an acid-activated montmorillonite.

2. A method, according to claim 1, wherein the method comprises the following steps;
a) providing the (meth)acrylate;
b) mixing the (meth)acrylate with the clay;
c) optionally diluting the mixture of (meth)acrylate and the clay with a solvent;
d) treating the (meth)acrylate by leaving it in contact with the clay for a sufficient time;
e) separating the treated (meth)acrylate from the clay by filtration;
f) optionally eliminating the solvent from the treated (meth)acrylate; and
g) optionally storing the treated (meth)acrylate.

3. A method, according to claim 1, wherein the method comprises the following steps;
a') providing the (meth)acrylate;
b') optionally diluting the (meth)acrylate with a solvent;
c') treating the (meth)acrylate by filtrating it through at least one layer of the clay;
d') optionally eliminating the solvent from the treated (meth)acrylate; and
e') optionally storing the treated (meth)acrylate.

4. A method, according to anyone of claims 1 to 3, wherein the acid-activated montmorillonite is obtained from a natural montmorillonite having a 2:1 layer mineral and having two silica tetrahedral sheets bonded to a central alumina octahedral sheet

5. A method, according to anyone of claims 1 to 4, wherein the acid-activated montmorillonite has a surface area BET from 150 to 380 m²g⁻¹.

6. A method, according to anyone of claims 1 to 5, wherein the acid-activated montmorillonite has a pore volume distribution (0-80 nm) from 0.20 to 0.55 ml. g⁻¹, a pore volume (0-24 nm) from 0.18 to 0.49 ml. g⁻¹, a pore volume (0-14 nm) from 0.13 to 0.43 ml. g⁻¹.

7. A method, according to anyone of claims 1 to 6, wherein the acid-activated montmorillonite has a S_{BET} from 150 to 380 m².g-¹, a number of sites from 0.13 to 0.32 mmol.g⁻¹, a number of sites from 0.50 to 1.40 µmol.m⁻².

8. A method, according to anyone of claims 1 to 7, wherein the (meth)acrylate comprises is selected from the group consisting of (meth)acrylic monomers, (meth)acrylic oligomers, and their mixtures thereof.

9. A method, according to anyone of claims 1 to 8, wherein the (meth)acrylate is selected from the group consisting of (meth)acrylate esters of aliphatic mono-alcohols, (meth)acrylate esters of alkoxylated aliphatic mono-alcohols, (meth)acrylate esters of aliphatic polyols, (meth)acrylate esters of alkoxylated aliphatic polyols, (meth)acrylate esters of aromatic ring-containing alcohols, (meth)acrylate esters of alkoxylated aromatic ring-containing alcohols, epoxy (meth)acrylates, polyether (meth)acrylates, urethane (meth)acrylates, polycarbonate (meth)acrylate), polyester (meth)acrylates, and mixtures thereof.

10. A method, according to anyone of claims 1 to 9, wherein the (meth)acrylate is selected from the group consisting of : 1,3-butylene glycol di(meth)acrylate, 1,4-butanediol di(meth)acrylate, 1,6-hexanediol di(meth)acrylate, longer chain aliphatic di(meth)acrylates (such as those generally corresponding to the formula H₂C=CRC(=O)-O-(CH₂)ₘ-O-C(=O)CR'=CH₂, wherein R and R' are independently H or methyl and m is an integer of 8 to 24), alkoxylated (e.g., ethoxylated, propoxylated) hexanediol di(meth)acrylates, alkoxylated (e.g., ethoxylated, propoxylated) neopentyl glycol di(meth)acrylates, dodecyl di(meth) acrylates, cyclohexane dimethanol di(meth)acrylates, diethylene glycol di(meth)acrylates, dipropylene glycol di(meth)acrylates, alkoxylated (e.g., ethoxylated, propoxylated) bisphenol A di(meth)acrylates, ethylene glycol di(meth)acrylates, neopentyl glycol di(meth)acrylates, tricyclodecane dimethanol diacrylates, triethylene glycol di(meth)acrylates, tetraethylene glycol di(meth)acrylates, tripropylene glycol di(meth)acrylates, ditrimethylolpropane tetra(meth)acrylates, dipentaerythritol penta(meth)acrylates, alkoxylated (e.g., ethoxylated, propoxylated) pentaerythritol tetra(meth)acrylate, dipentaerythritol penta(meth)acrylates, pentaerythritol tetra(meth)acrylate, alkoxylated (e.g., ethoxylated, propoxylated) trimethylolpropane tri(meth)acrylates, alkoxylated (e.g., ethoxylated, propoxylated) glyceryl tri(meth)acrylates, trimethylolpropane tri(meth)acrylates, pentaerythritol tri(meth)acrylates, tris (2-hydroxy ethyl) isocyanurate tri(meth)acrylates, 2(2-ethoxyethoxy) ethyl (meth)acrylates, 2-phenoxyethyl (meth)acrylates, 3,3,5-trimethylcyclohexyl (meth)acrylates, alkoxylated lauryl (meth)acrylates, alkoxylated phenol (meth)acrylates, alkoxylated tetrahydrofurfuryl (meth)acrylates, caprolactone (meth)acrylates, cyclic trimethylolpropane formal (meth)acrylates, dicyclopentadienyl (meth)acrylates, diethylene glycol methyl ether (meth)acrylates, alkoxylated (e.g., ethoxylated, propoxylated) nonyl phenol (meth)acrylates, isobornyl (meth)acrylates, isodecyl (meth)acrylates, isooctyl (meth)acrylates, lauryl (meth)acrylates, methoxy polyethylene glycol (meth)acrylates, octyldecyl (meth)acrylates (also known as stearyl (meth)acrylates), tetrahydrofurfuryl (meth) acrylates, tridecyl (meth)acrylates, triethylene glycol ethyl ether (meth)acrylates, t-butyl cyclohexyl (meth)acrylates, dicyclopentadiene di(meth)acrylates, phenoxyethanol (meth)acrylates, octyl (meth)acrylates, decyl (meth)acrylates, dodecyl (meth)acrylates, tetradecyl (meth)acrylates, cetyl (meth)acrylates, hexadecyl (meth)acrylates, behenyl (meth)acrylates, diethylene glycol ethyl ether (meth)acrylates, diethylene glycol butyl ether (meth)acrylates, triethylene glycol methyl ether (meth)acrylates, dodecanediol di (meth)acrylates, dipentaerythritol penta/hexa(meth)acrylates, pentaerythritol tetra(meth)acrylates, alkoxylated (e.g., ethoxylated, propoxylated) pentaerythritol tetra(meth)acrylates, di-trimethylolpropane tetra(meth)acrylates, alkoxylated (e.g., ethoxylated, propoxylated) glyceryl tri(meth)acrylates, and tris (2-hydroxy ethyl) isocyanurate tri(meth)acrylates, and combinations thereof.

11. A method, according to anyone of claims 1 to 8, wherein the (meth)acrylate is selected from the group consisting of oligomers such as epoxy (meth)acrylates, polyether (meth)acrylates, urethane (meth)acrylates, polycarbonate (meth)acrylate), polyester (meth)acrylates (including amine- and sulfide-modified derivatives thereof), and mixtures thereof.

12. A method, according to anyone of claims 1 to 11, wherein the (meth)acrylate does not comprise a cyano group.

13. A method, according to anyone of claims 1 to 12, wherein the solvent is a non-nucleophilic solvent, preferably selected from the group consisting of aliphatic and aromatic hydrocarbons, ether, ester, ketone, nitrile, and mixtures thereof.

14. A (meth)acrylate obtained by the method, according to anyone of claims 1 to 13, wherein the (meth)acrylate has a content of metal and metalloid atoms, lower than or equal to 200 ppm.

15. A (meth)acrylate according to anyone of claim 14, wherein the (meth)acrylate comprises less than 50 ppm of tin (Sn).

16. Use of the (meth)acrylate according to anyone of claims 14 or 15, in different applications, including in 3D printing, coating, sealant, adhesives, and electronics.

17. Curable composition comprising the (meth)acrylate according to anyone of claims 14 to 16.

18. Use of a clay being an acid-activated montmorillonite for treating a (meth)acrylate.
